# EUROPEAN PATENT SPECIFICATION

(11) **EP 3 705 096 B1**
(45) Date of publication and mention of the grant of the patent: **29.09.2021**
(21) Application number: 19175174.2
(22) Date of filing: 17.05.2019
(51) Int. Cl.: A61F 11/08, A61F 11/10

(54) **EARPLUG WITH ENHANCED WEARING COMFORTABILITY**
OHRSTÖPSEL MIT ERHÖHTEM TRAGEKOMFORT
BOUCHON D'OREILLE AYANT UN MEILLEUR CONFORT DE PORT

(30) Priority: 05.03.2019 TW 108107177
(43) Date of publication of application: 09.09.2020
(73) Proprietor: DRAGONSTATE TECHNOLOGY CO., LTD., Taiwan (TW)
(72) Inventor: CHEN, Jie-Ru, New Taipei City 23674 (TW); TSAI, Tzu-Ching, New Taipei City 23674 (TW)
(74) Representative: Lang, Christian

(56) References cited:
- DE-A1-102014 221 590
- GB-A- 1 274 274
- US-B2- 6 820 717

## Description

### BACKGROUND OF THE INVENTION

### (a) Field of the Invention

The present invention relates to an earplug, and more particularly to an earplug 10, 10' that is inserted and worn inside a person's ear canal 60, and uses circulation of air to enhance comfortability when wearing.

### (b) Description of the Prior Art

Earplugs are mainly used for placing inside a person's ear canal to completely cut off noise from entering the ear to achieve protection of the person's ear canal.

Commonly seen earplugs products mainly use an integral body formed from foam or silicone to produce a multi-disc umbrella shaped body that facilitates placement inside a person's ear canal to achieve a protection effect. However, after insertion into the ear canal for a long period of time, these types of earplug products easily result in discomfort in the person's ear canal, and is unable to satisfy various different types of ear canals.

Hence, presently, various different inflatable earplug products have appeared on the market, such as U.S. Patent No. 20090173353 or, U.S. Patent No. 2015282990, These types of inflatable ear protector products directly fill bags with air, however, after inflating, these types of inflatable ear protector products do not allow separate adjustment of the inflation state of the bags, which easily causes discomfort when wearing the ear protector.

U.S. Patent No. 9326059 discloses another type of inflatable ear protector product, which is configured with a plurality of bags that are assembled and mutually connected using an encircling center method. When inflating the ear protector, the plurality of bags are concurrently inflated, however, during inflation, this type of inflatable ear protector product similarly disallows the separate adjustment of the inflation state of the plurality of bags, which not only easily causes discomfort when wearing the ear protector, but is also unsuitable for various different types of ear canals, and thus unable to satisfy various different ear canals, resulting in poor application effectiveness. Document GB-A-1274274 discloses the most relevant prior art.

Accordingly, the technical problems the present invention intends to resolve and objectives thereof involves providing an earplug with enhanced wearing comfortability.

### SUMMARY OF THE INVENTION

The main object of the present invention lies in providing an earplug, and more particularly to an earplug that is inserted and worn inside a person's ear canal, and uses circulation of air to enhance comfortability when wearing.

In order to achieve the aforementioned object, an earplug with enhanced wearing comfortability of the present invention comprises a main guide body, a supporting stem, and a filler material provided with plasticity. The main guide body further comprises a guide portion, a compression portion that extends from the guide portion, and a stop portion that extends from the compression portion. A connecting cavity is provided inside the guide portion, a filling cavity is provided inside the compression portion, and an opening portion is provided inside the stop portion. The filling cavity interconnects the connecting cavity and the opening portion. The supporting stem is provided with a connecting section that enables embedding into the connecting cavity, an extended section that extends from the connecting section and is positioned inside the filling cavity, and a securing section that extends from the extended section and embeds within the opening portion. Moreover, the securing section is provided with an air passage, which enables air to circulate between the exterior of the main guide body and the interior of the filling cavity by passing through the air passage. The filler material fills the interior of the filling cavity to enable an air chamber that can contain air to form therein, thereby enabling air to enter the air chamber through the air passage for the compression portion to maintain its original state. Accordingly, when the compression portion is pressed and constricted, the compression portion compresses the air within the air chamber through the filler material, thereby expelling the air through the air passage and causing the compression portion to form a compressed state. After releasing the pressure on the compression portion, air enters the air chamber through the air passage and squeezes the filler material, which causes the compression portion to return to its original state from a compressed state.

In an embodiment, the compression portion is further assembled from a first compression area connected to the guide portion and a second compression area that extends from the first compression area and is connected to the stop portion.

In the above mentioned embodiment, the filling cavity is further is assembled from a first filling cavity that is positioned inside the first compression area and a second filling cavity that is positioned inside the second compression area and connects with the air passage; moreover, the first filling cavity and the second filling cavity are interconnected.

In the above mentioned embodiment, the filler material fills the first filling cavity and a portion of the second filling cavity, and the air chamber is formed inside the second filling cavity.

In the embodiments, a mutually embedding structure is formed between the opening portion and the securing section.

In the embodiments, the embedding structure comprises a first embedding portion positioned at the opening portion and a second embedding portion positioned on the securing section and embedded into the first embedding portion.

In the embodiments, both the guide portion and the stop portion assume an umbrella form.

In the embodiments, the filler material is formed from silicone material.

In the embodiments, the supporting stem further comprises a holding section extending from the securing section to the outer side of the opening portion, and the holding section extends to the exterior of the main guide body.

The earplug of the present invention has the following advantages compared to the prior art:
When inserting the earplug into a person's ear canal, the compression portion compresses in accordance with changes in the shape of the person's ear canal, and the shape of the compression portion changes through air adjustment to achieve enhanced comfortability when wearing the earplug.

To enable a further understanding of said objectives and the technological methods of the invention herein, a brief description of the drawings is provided below followed by a detailed description of the preferred embodiments.

### BRIEF DESCRIPTION OF THE DRAWINGS

Figure 1 is a cross-sectional schematic view of a first embodiment aspect of the present invention.
Figure 2 is a three-dimensional view of a second embodiment aspect of the present invention.
Figure 3 is planar schematic view of the second embodiment aspect of the present invention.
Figure 4 is a cross-sectional schematic view of a first viewing angle of the second embodiment of the present invention.
Figure 5 is a cross-sectional schematic view of a second viewing angle of the second embodiment aspect of the present invention.
Figure 6 is a cross-sectional schematic view of a decomposed state of the first viewing angle of the second embodiment aspect of the present invention.
Figure 7 is schematic view of the second embodiment of the present invention placed inside a person's ear canal.

### DETAILED DESCRIPTION OF THE PREFERRED EMBODIMENTS

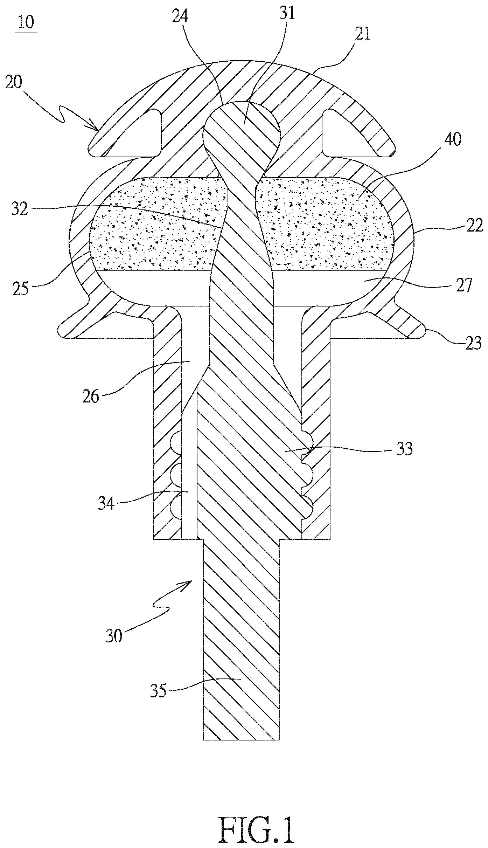

Regarding the detailed content and structural means of present invention, please refer to the description of the diagrams as follows:
Referring to Figure 1, which shows an earplug with enhanced wearing comfortability of the present invention, wherein an earplug 10 comprises a main guide body 20, a supporting stem 30, and a filler material 40.

The main guide body 20 further comprises a guide portion 21, a compression portion 22 that extends from the guide portion 21, and a stop portion 23 that extends from the compression portion 22, all arranged in an axial configuration. The exterior of the guide portion 21 assumes an umbrella form, and a connecting cavity 24 is provided inside the guide portion 21. A filling cavity 25 is provided inside the compression portion 22, the exterior of the stop portion 23 assumes an umbrella form, and an opening portion 26 is provided inside the stop portion 23. The filling cavity 25 interconnects the connecting cavity 24 and the opening portion 26.

The supporting stem 30 that extends along the same axial direction as the main guide body 20 is provided with a connecting section 31 that enables embedding into the connecting cavity 24, an extended section 32 that extends from the connecting section 31 and is positioned inside the filling cavity 25, and a securing section 33 that extends from the extended section 32 and embeds within the opening portion 26. Moreover, the securing section 33 is provided with an air passage 34, which enables air to pass therethrough (not shown in the drawing) and circulate between the exterior of the main guide body 20 and the interior of the filling cavity 25. In addition, a holding section 35 further extends outward to the exterior of the main guide body 20 from the securing section 33.

The filler material 40 is provided with plasticity and fills the interior of the filling cavity 25 to enable an air chamber 27 to form therein and contain air. Air can enter the air chamber 27 through the air passage 34 for the compression portion 22 to maintain an original state.

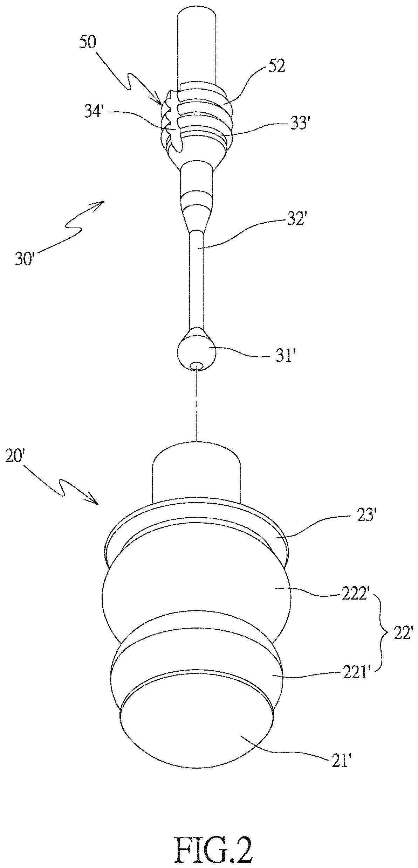

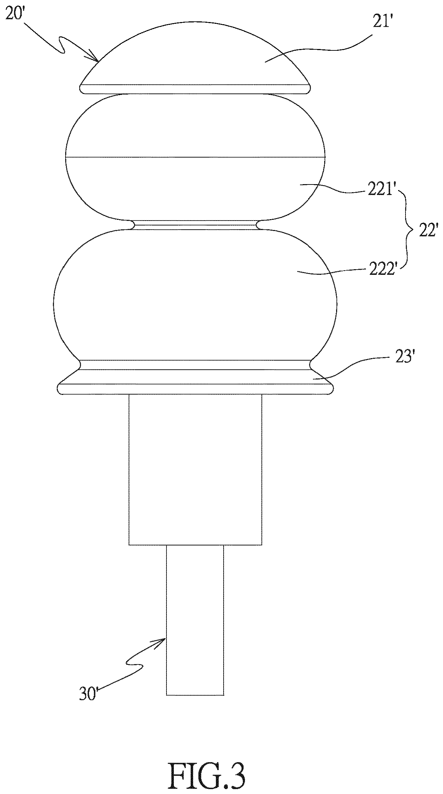

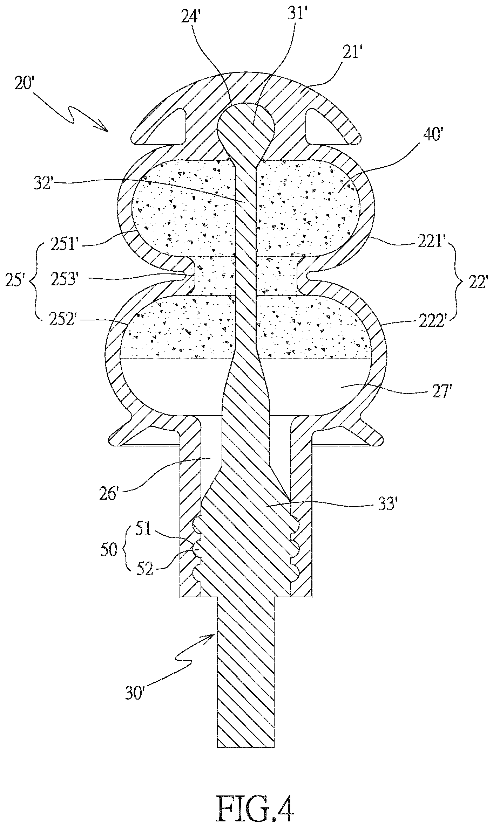

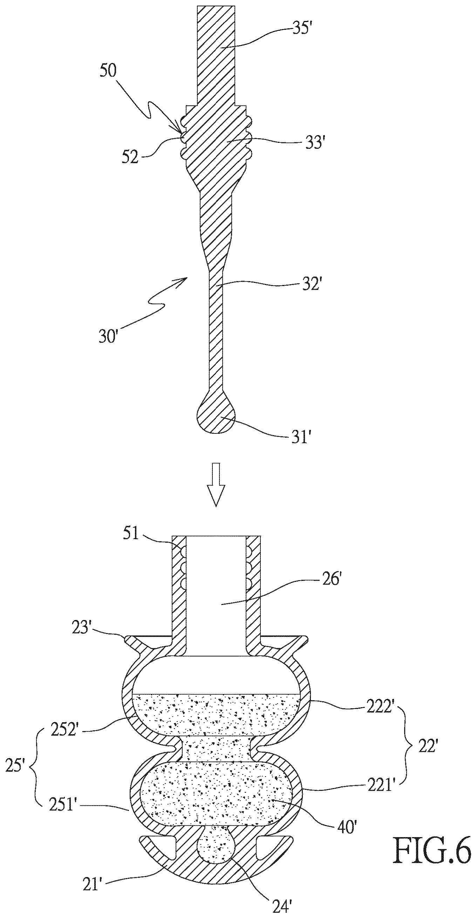

Referring further to Figures 2 to 6, which show a second embodiment aspect of the earplug 10, and in this embodiment a compression portion 22' further comprises a first compression area 221' that extends from a guide portion 21' and a second compression area 222' that extends from the first compression area 221'.

The second compression area 222' is connected to a stop portion 23', and a filling cavity 25' further comprises a first filling cavity 251' that is positioned inside the first compression area 221' and a second filling cavity 252' that is positioned inside the second compression area 222', wherein a passage cavity 253' provides interconnection between the first filling cavity 251' and the second filling cavity 252'. The diameter of the passage cavity 253' is smaller than the diameters of both the first filling cavity 251' and the second filling cavity 252', and the diameter of the first filling cavity 251' is slightly smaller than that of the second filling cavity 252'. The second filling cavity 252' is connected to an opening portion 26', and a filler material 40' respectively fills the first filling cavity 251', the passage cavity 253', and the interior of the second filling cavity 252'. Moreover, an air chamber 27' is positioned inside the second filling cavity 252' and connects to an air passage 34'.

It is worth mentioning that the filler material 40' in the second embodiment is formed from silicone material, and the silicone filler material 40' is first heated to form a liquid, after which the liquid silicone material is injected into the interior of the main guide body 20'. It is important to explain that the liquid silicone material does not completely fill the interior of the second filling cavity 252' when injecting the liquid silicone material into the interior of the main guide body 20'. After injecting the liquid silicone material, the supporting stem 30' is disposed into the interior of the main guide body 20' before the liquid silicone material has cooled down to enable inserting and embedding a connecting section 31' of the supporting stem 30' into a connecting cavity 24' while enabling an extended section 32' to extend into the filling cavity 25'.

In addition, an embedding structure 50 is further configured between a securing section 33' and the opening portion 26', wherein the embedding structure 50 comprises: a first embedding portion 51 positioned at the opening portion 26' and a second embedding portion 52 positioned on the securing section 33' and embedded into the first embedding portion 51. Accordingly, when the supporting stem 30' is disposed into the interior of the main guide body 20', apart from embedding the connecting section 31' into the connecting cavity 24', the supporting stem 30' is also embedded into the opening portion 26' by means of the securing section 33', thereby enabling the supporting stem 30' to effectively embed into the main guide body 20' and preventing separation from occurring between the supporting stem 30' and the main guide body 20'.

After disposing the supporting stem 30' into the interior of the main guide body 20', a silicone material is caused to solidify inside the filling cavity 25' and form the filler material 40' using a cooling or photocuring method, which also causes the air chamber 27' to form within the second filling cavity 252', thereby enabling air to pass through the air passage 34' on the supporting stem 30' from the exterior of the main guide body 20' and enter the air chamber 27', which further enables the compression portion 22' to maintain its original state.

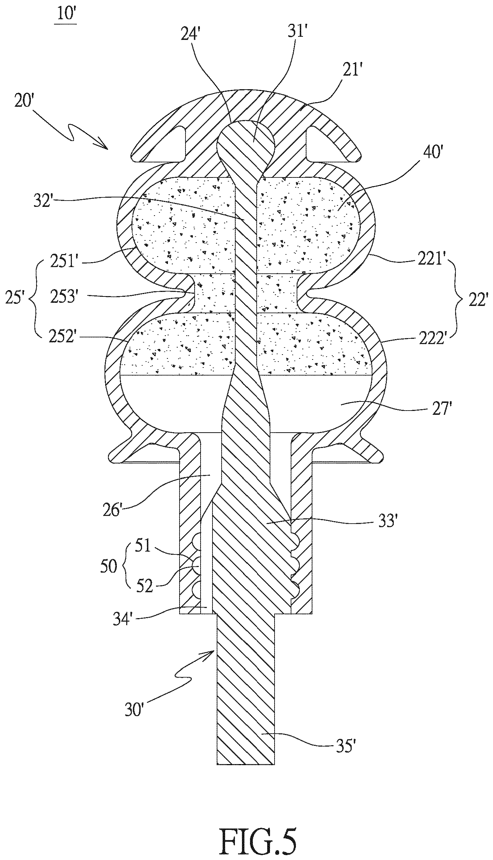

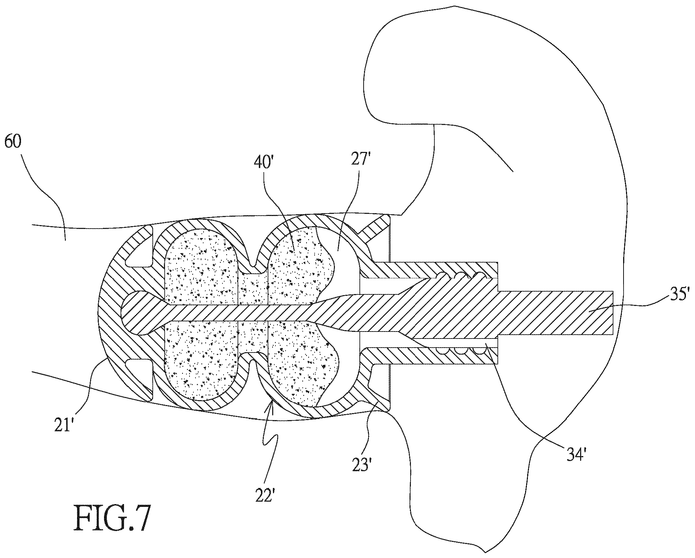

Referring to Figures 5 to 7, when an earplug 10' is inserted inside a person's ear canal 60, it is of prime importance to first use the guide portion 21' to enter the person's ear canal 60, which is followed by the compression portion 22' gradually coming into contact with the interior of the person's ear canal 60, whereupon the ear wall presses against the compression portion 22' causing deformation thereof and compression of the filler material 40' within the filling cavity 25'. And because the filler material 40' is provided with plasticity, thus, the filler material 40' also deforms within the filling cavity 25'. After deforming, the filler material 40' produces a pushing force toward the air chamber 27' that squeezes and expels the air therein to the exterior of the main guide body 20' through the air passage 34', thereby causing the compression portion 22' to form a compressed state and enabling the stop portion 23' to block the entrance area of the person's ear canal 60. Accordingly, apart from enabling the compression portion 22' to produce a more favorable contact effect with the person's ear canal 60, the earplug 10' achieves better airtightness within the person's ear canal 60. In addition, compression of the compression portion 22' by the person's ear canal 60 compresses the filler material 40', which then squeezes and expels the air out to effectively reduce the pressure within the earplug 10', thereby enhancing comfortability when wearing the earplug 10'.

On the contrary, when the earplug 10' is gradually separated from the person's ear canal 60, the pressure produced by the ear wall on the compression portion 22' also gradually decreases along with a correspondingly gradual decrease in the pushing force of the filler material 40' on the air, at which time the pressure within the air chamber 27' is less than the atmospheric pressure outside the person's ear canal 60, which causes air to gradually enter the air chamber 27' through the air passage 34' and compress the filler material 40' until the earplug 10' is completely separated from the person's ear canal 60, whereupon the filler material 40' continues to be compressed by the air, causing the compression portion 22' to return to its original state from a compressed state, thereby further providing the earplug 10' with the effect of reusability and substantially extending the serviceable life thereof.

It is worth mentioning that it is of prime importance to hold a holding section 35' of the supporting stem 30' when inserting/extracting the earplug 10' into/out of the person's ear canal 60 to ensure added convenience when carrying out insertion or extraction of the earplug 10'.

## Claims

1. An earplug with enhanced wearing comfortability, comprising:
a main guide body (20, 20'), provided with a guide portion (21, 21'), a compression portion (22, 22') that extends from the guide portion (21, 21'), and a stop portion (23, 23') that extends from the compression portion (22, 22'), wherein a connecting cavity (24, 24') is provided inside the guide portion (21, 21'), a filling cavity (25, 25') is provided inside the compression portion (22, 22'), and an opening portion (26, 26') is provided inside the stop portion (23, 23'); moreover, the filling cavity (25, 25') interconnects the connecting cavity (24, 24') and the opening portion (26, 26');
a supporting stem (30, 30'), which is provided with a connecting section (31, 31') that enables embedding into the connecting cavity (24, 24'), an extended section (32, 32') that extends from the connecting section (31, 31') and is positioned inside the filling cavity (25, 25'), and a securing section (33, 33') that extends from the extended section (32, 32') and embeds within the opening portion (26, 26'); moreover, the securing section (33, 33') is provided with an air passage (34, 34'), which enables air to pass therethrough from an exterior of the main guide body (20, 20') and circulate to an interior of the filling cavity (25, 25'); and
a filler material (40, 40') provided with plasticity, which fills the interior of the filling cavity (25, 25') and causes an air chamber (27, 27') to form therein and contain air, such that, air enters the air chamber (27, 27') through the air passage (34, 34') and cause the compression portion (22, 22') to form its original state;
whereby when the compression portion (22, 22') is pressed and constricted, the compression portion (22, 22') compresses the air within the air chamber (27, 27') through the filler material (40, 40'), thereby expelling the air through the air passage (34, 34') and causing the compression portion (22, 22') to form a compressed state; and after releasing the pressure on the compression portion (22, 22'), air enters the air chamber (27, 27') through the air passage (34, 34') and squeezes the filler material (40, 40'), thereby causing the compression portion (22, 22') to return to an original state from a compressed state.

2. The earplug with enhanced wearing comfortability according to claim 1, wherein the compression portion (22') is further assembled from a first compression area (221') connected to the guide portion (21') and a second compression area (222') that extends from the first compression area (221') and connects with the stop portion (23').

3. The earplug with enhanced wearing comfortability according to claim 2, wherein the filling cavity (25') is further assembled from a first filling cavity (251') that is positioned inside the first compression area (221') and a second filling cavity (252') that is positioned inside the second compression area (222') and connects with the air passage (34'); moreover, the first filling cavity (251') and the second filling cavity (252') are interconnected.

4. The earplug with enhanced wearing comfortability according to claim 3, wherein the filler material (40') fills the first filling cavity (252') and a portion of the second filling cavity (252'), thereby causing the air chamber (27') to form inside the second filling cavity (252').

5. The earplug with enhanced wearing comfortability according to claim 1, wherein a mutually embedding structure (50) is formed between the opening portion (26, 26') and the securing section (33, 33').

6. The earplug with enhanced wearing comfortability according to claim 5, wherein the embedding structure (50) comprises a first embedding portion (51) positioned at the opening portion (26, 26') and a second embedding portion (52) positioned on the securing section (33, 33') and embeds into the first embedding portion (51).

7. The earplug with enhanced wearing comfortability according to claim 1, wherein both the guide portion (21, 21') and the stop portion (23, 23') assume an umbrella form.

8. The earplug with enhanced wearing comfortability according to claim 1, wherein the filler material (40, 40') is formed from silicone material.

9. The earplug with enhanced wearing comfortability according to claim 1, wherein the supporting stem (30, 30') further comprises a holding section (35, 35') extending from the securing section (33, 33') toward an outer side of the opening portion (26, 26'), and the holding section (35, 35') extends to the exterior of the main guide body (20, 20').

## Patentansprüche

1. Ohrstöpsel mit verbessertem Tragekomfort, umfassend:
einen Hauptführungskörper (20, 20'), versehen mit einem Führungsabschnitt (21, 21'), einem Kompressionsabschnitt (22, 22'), der sich von dem Führungsabschnitt (21, 21') erstreckt, und einem Stopabschnitt (23, 23'), der sich von dem Kompressionsabschnitt (22, 22') erstreckt, wobei ein Verbindungshohlraum (24, 24') innerhalb des Führungsabschnitts (21, 21'), ein Füllhohlraum (25, 25') innerhalb des Kompressionsabschnitts (22, 22') und ein Öffnungsabschnitt (26, 26') innerhalb des Stopabschnitts (23, 23') vorgesehen ist, wobei darüber hinaus der Füllhohlraum (25, 25') den Verbindungshohlraum (24, 24') und den Öffnungsabschnitt (26, 26') miteinander verbindet,
einen Stützschaft (30, 30'), der mit einem Verbindungsabschnitt (31, 31'), der ein Einbetten in den Verbindungshohlraum (24, 24') ermöglicht, einem Verlängerungsabschnitt (32, 32'), der sich von dem Verbindungsabschnitt (31, 31') erstreckt und innerhalb des Füllhohlraums (25, 25') positioniert ist, und einem Befestigungsabschnitt (33, 33') versehen ist, der sich von dem Verlängerungsabschnitt (32, 32') erstreckt und in den Öffnungsabschnitt (26, 26') eingebettet ist,
wobei ferner der Befestigungsabschnitt (33, 33') mit einem Luftdurchgang (34, 34') versehen ist, der es ermöglicht, dass Luft von einer Außenseite des Hauptführungskörpers (20, 20') hindurchtritt und zu einem Inneren des Füllhohlraums (25, 25') zirkuliert, und
ein mit Plastizität versehenes Füllmaterial (40, 40'), das das Innere des Füllhohlraums (25, 25') füllt und bewirkt, dass sich darin eine Luftkammer (27, 27') bildet und Luft enthält, so dass Luft durch den Luftdurchgang (34, 34') in die Luftkammer (27, 27') eintritt und bewirkt, dass der Kompressionsabschnitt (22, 22') seinen ursprünglichen Zustand bildet,
wodurch, wenn der Kompressionsabschnitt (22, 22') gedrückt und eingeengt wird, der Kompressionsabschnitt (22, 22') die Luft innerhalb der Luftkammer (27, 27') durch das Füllmaterial (40, 40') komprimiert, wodurch die Luft durch den Luftdurchgang (34, 34') ausgestoßen wird und der Kompressionsabschnitt (22, 22') veranlasst wird, einen komprimierten Zustand zu bilden, und wobei nach dem Ablassen des Drucks auf den Kompressionsabschnitt (22, 22') Luft durch den Luftdurchgang (34, 34') in die Luftkammer (27, 27') eintritt und das Füllmaterial (40, 40') zusammendrückt, wodurch der Kompressionsabschnitt (22, 22') aus einem komprimierten Zustand in einen ursprünglichen Zustand zurückkehrt.

2. Ohrstöpsel mit erhöhtem Tragekomfort nach Anspruch 1, bei welchem der Kompressionsabschnitt (22') weiterhin aus einem ersten Kompressionsbereich (221'), der mit dem Führungsabschnitt (21') verbunden ist, und einem zweiten Kompressionsbereich (222'), der sich von dem ersten Kompressionsbereich (221') erstreckt und mit dem Stopabschnitt (23') verbunden ist, zusammengesetzt ist.

3. Ohrstöpsel mit erhöhtem Tragekomfort nach Anspruch 2, bei welchem der Füllhohlraum (25') weiterhin aus einem ersten Füllhohlraum (251'), der innerhalb des ersten Kompressionsbereichs (221') angeordnet ist, und einem zweiten Füllhohlraum (252'), der innerhalb des zweiten Kompressionsbereichs (222') angeordnet ist und mit dem Luftdurchgang (34') verbunden ist, zusammengesetzt ist, wobei außerdem der erste Füllhohlraum (251') und der zweite Füllhohlraum (252') miteinander verbunden sind.

4. Ohrstöpsel mit erhöhtem Tragekomfort nach Anspruch 3, bei welchem das Füllmaterial (40') den ersten Füllhohlraum (252') und einen Teil des zweiten Füllhohlraums (252') ausfüllt, wodurch sich die Luftkammer (27') innerhalb des zweiten Füllhohlraums (252') bildet.

5. Ohrstöpsel mit erhöhtem Tragekomfort nach Anspruch 1, bei welchem zwischen dem Öffnungsabschnitt (26, 26') und dem Befestigungsabschnitt (33, 33') eine wechselseitig eingebettete Struktur (50) ausgebildet ist.

6. Ohrstöpsel mit erhöhtem Tragekomfort nach Anspruch 5, bei welchem die eingebettete Struktur (50) einen ersten Einbettungsabschnitt (51), der an dem Öffnungsabschnitt (26, 26') angeordnet ist, und einen zweiten Einbettungsabschnitt (52), der an dem Befestigungsabschnitt (33, 33') angeordnet ist und in den ersten Einbettungsabschnitt (51) eingebettet ist, aufweist.

7. Ohrstöpsel mit erhöhtem Tragekomfort nach Anspruch 1, bei welchem sowohl der Führungsabschnitt (21, 21') als auch der Stopabschnitt (23, 23') eine Schirmform annehmen.

8. Ohrstöpsel mit erhöhtem Tragekomfort nach Anspruch 1, bei welchem das Füllmaterial (40, 40') aus Silikonmaterial gebildet ist.

9. Ohrstöpsel mit erhöhtem Tragekomfort nach Anspruch 1, bei welchem der Stützschaft (30, 30') ferner einen Halteabschnitt (35, 35') aufweist, der sich von dem Befestigungsabschnitt (33, 33') zu einer Außenseite des Öffnungsabschnitts (26, 26') erstreckt, und der Halteabschnitt (35, 35') sich zur Außenseite des Hauptführungskörpers (20, 20') erstreckt.

## Revendications

1. Bouchon d'oreille avec un meilleur confort de port, comprenant:
un corps de guidage principal (20, 20'), muni d'une partie de guidage (21, 21'), d'une partie de compression (22, 22') qui s'étend à partir de la partie de guidage (21, 21'), et d'une partie d'arrêt (23, 23') qui s'étend à partir de la partie de compression (22, 22'), dans lequel une cavité de connexion (24, 24') est prévue à l'intérieur de la partie de guidage (21, 21'), une cavité de remplissage (25, 25') est prévue à l'intérieur de la partie de compression (22, 22'), et une partie d'ouverture (26, 26') est prévue à l'intérieur de la partie d'arrêt (23, 23'); de plus, la cavité de remplissage (25, 25') interconnecte la cavité de connexion (24, 24') et la partie d'ouverture (26, 26');
une tige de support (30, 30'), qui est munie d'une section de connexion (31, 31') qui permet un encastrement dans la cavité de connexion (24, 24'), d'une section étendue (32, 32') qui s'étend à partir de la section de connexion (31, 31') et est positionnée à l'intérieur de la cavité de remplissage (25, 25'), et d'une section de fixation (33, 33') qui s'étend à partir de la section étendue (32, 32') et s'encastre à l'intérieur de la partie d'ouverture (26, 26'); de plus, la section de fixation (33, 33') est munie d'un passage d'air (34, 34'), qui permet à de l'air de passer à travers celui-ci à partir d'un extérieur du corps de guidage principal (20, 20') et de circuler vers un intérieur de la cavité de remplissage (25, 25'); et
un matériau de remplissage (40, 40') muni d'une plasticité, qui remplit l'intérieur de la cavité de remplissage (25, 25') et amène une chambre d'air (27, 27') à se former dans celle-ci et à contenir de l'air, de telle sorte que de l'air pénètre dans la chambre d'air (27, 27') par le passage d'air (34, 34') et amène la partie de compression (22, 22') à former son état d'origine;
moyennant quoi lorsque la partie de compression (22, 22') est pressée et rétrécie, la partie de compression (22, 22') comprime l'air à l'intérieur de la chambre d'air (27, 27') à travers le matériau de remplissage (40, 40'), en expulsant ainsi l'air à travers le passage d'air (34, 34') et en amenant la partie de compression (22, 22') à former un état comprimé; et après libération de la pression sur la partie de compression (22, 22'), de l'air pénètre dans la chambre d'air (27, 27') à travers le passage d'air (34, 34') et presse le matériau de remplissage (40, 40'), en amenant ainsi la partie de compression (22, 22') à revenir à un état d'origine à partir d'un état comprimé.

2. Bouchon d'oreille avec un meilleur confort de port selon la revendication 1, dans lequel la partie de compression (22') est en outre assemblée à partir d'une première zone de compression (221') connectée à la partie de guidage (21') et d'une seconde zone de compression (222') qui s'étend à partir de la première zone de compression (221') et se connecte à la partie d'arrêt (23').

3. Bouchon d'oreille avec un meilleur confort de port selon la revendication 2, dans lequel la cavité de remplissage (25') est en outre assemblée à partir d'une première cavité de remplissage (251') qui est positionnée à l'intérieur de la première zone de compression (221') et d'une seconde cavité de remplissage (252') qui est positionnée à l'intérieur de la seconde zone de compression (222') et se connecte au passage d'air (34'); de plus, la première cavité de remplissage (251') et la seconde cavité de remplissage (252') sont interconnectées.

4. Bouchon d'oreille avec un meilleur confort de port selon la revendication 3, dans lequel le matériau de remplissage (40') remplit la première cavité de remplissage (252') et une partie de la seconde cavité de remplissage (252'), en amenant ainsi la chambre d'air (27') à se former à l'intérieur de la seconde cavité de remplissage (252').

5. Bouchon d'oreille avec un meilleur confort de port selon la revendication 1, dans lequel une structure d'encastrement mutuel (50) est formée entre la partie d'ouverture (26, 26') et la section de fixation (33, 33').

6. Bouchon d'oreille avec un meilleur confort de port selon la revendication 5, dans lequel la structure d'encastrement (50) comprend une première partie d'encastrement (51) positionnée au niveau de la partie d'ouverture (26, 26') et une seconde partie d'encastrement (52) positionnée sur la section de fixation (33, 33') et encastrée dans la première partie d'encastrement (51).

7. Bouchon d'oreille avec un meilleur confort de port selon la revendication 1, dans lequel à la fois la partie de guidage (21, 21') et la partie d'arrêt (23, 23') prennent une forme de parapluie.

8. Bouchon d'oreille avec un meilleur confort de port selon la revendication 1, dans lequel le matériau de remplissage (40, 40') est formé à partir d'un matériau de silicone.

9. Bouchon d'oreille avec un meilleur confort de port selon la revendication 1, dans lequel la tige de support (30, 30') comprend en outre une section de maintien (35, 35') s'étendant à partir de la section de fixation (33, 33') vers un côté extérieur de la partie d'ouverture (26, 26'), et la section de maintien (35, 35') s'étend vers l'extérieur du corps de guidage principal (20, 20').
